# EUROPEAN PATENT APPLICATION

(11) **EP 3 633 372 A1**
(43) Date of publication of application: **08.04.2020**
(21) Application number: 18809506.1
(22) Date of filing: 16.05.2018
(51) Int. Cl.: G01N 33/53, C07K 16/18, G01N 33/68

(54) **BIOMARKER FOR ALZHEIMER'S DISEASE**

(30) Priority: 31.05.2017 JP 2017108766
(71) Applicant: Public University Corporation Nagoya City University, Nagoya-shi, Aichi 467-8601 (JP)
(72) Inventor: MICHIKAWA Makoto, Nagoya-shi Aichi 467-8601 (JP); AKATSU Hiroyasu, Nagoya-shi Aichi 467-8601 (JP); ABDULLAH Mohammad, Nagoya-shi Aichi 467-8601 (JP); MATSUBARA Etsuro, Yufu-shi Oita 879-5593 (JP); KIMURA Noriyuki, Yufu-shi Oita 879-5593 (JP)
(74) Representative: Steinecke, Peter
(86) International application number: PCT/JP2018/018849
(87) International publication number: WO 2018/221212

(57) **Abstract**

An object of the present invention is to provide a marker useful for early diagnosis and differentiation of Alzheimer's disease, and use thereof. An Alzheimer's disease biomarker composed of blood flotillin is provided.

### [Sequence Listing]

SEQUENCE LISTING

<110>  NAGOYA CITY UNIVERSITY
       NATIONAL UNIVERSITY CORPORATION OITA UNIVERSITY

<120>  BIOMARKER FOR ALZHEIMER'S DISEASE

<130>  NC17005P

<150>  JP P2017-108766
<151>  2017-05-31

<160>  1

<170>  PatentIn version 3.5

<210>  1
<211>  427
<212>  PRT
<213>  Homo sapiens

<400>  1

Met Phe Phe Thr Cys Gly Pro Asn Glu Ala Met Val Val Ser Gly Phe
1               5                   10                  15

Cys Arg Ser Pro Pro Val Met Val Ala Gly Gly Arg Val Phe Val Leu
            20                  25                  30

Pro Cys Ile Gln Gln Ile Gln Arg Ile Ser Leu Asn Thr Leu Thr Leu
        35                  40                  45

Asn Val Lys Ser Glu Lys Val Tyr Thr Arg His Gly Val Pro Ile Ser
    50                  55                  60

Val Thr Gly Ile Ala Gln Val Lys Ile Gln Gly Gln Asn Lys Glu Met
65                  70                  75                  80

Leu Ala Ala Ala Cys Gln Met Phe Leu Gly Lys Thr Glu Ala Glu Ile
                85                  90                  95

Ala His Ile Ala Leu Glu Thr Leu Glu Gly His Gln Arg Ala Ile Met
            100                 105                 110

Ala His Met Thr Val Glu Glu Ile Tyr Lys Asp Arg Gln Lys Phe Ser
        115                 120                 125

Glu Gln Val Phe Lys Val Ala Ser Ser Asp Leu Val Asn Met Gly Ile
    130                 135                 140

Ser Val Val Ser Tyr Thr Leu Lys Asp Ile His Asp Asp Gln Asp Tyr
145                 150                 155                 160

Leu His Ser Leu Gly Lys Ala Arg Thr Ala Gln Val Gln Lys Asp Ala
                165                 170                 175

Arg Ile Gly Glu Ala Glu Ala Lys Arg Asp Ala Gly Ile Arg Glu Ala
            180                 185                 190

Lys Ala Lys Gln Glu Lys Val Ser Ala Gln Tyr Leu Ser Glu Ile Glu
        195                 200                 205

Met Ala Lys Ala Gln Arg Asp Tyr Glu Leu Lys Lys Ala Ala Tyr Asp
    210                 215                 220

Ile Glu Val Asn Thr Arg Arg Ala Gln Ala Asp Leu Ala Tyr Gln Leu
225                 230                 235                 240

Gln Val Ala Lys Thr Lys Gln Gln Ile Glu Glu Gln Arg Val Gln Val
                245                 250                 255

Gln Val Val Glu Arg Ala Gln Gln Val Ala Val Gln Glu Gln Glu Ile
            260                 265                 270

Ala Arg Arg Glu Lys Glu Leu Glu Ala Arg Val Arg Lys Pro Ala Glu
        275                 280                 285

Ala Glu Arg Tyr Lys Leu Glu Arg Leu Ala Glu Ala Glu Lys Ser Gln
    290                 295                 300

Leu Ile Met Gln Ala Glu Ala Glu Ala Ala Ser Val Arg Met Arg Gly
305                 310                 315                 320

Glu Ala Glu Ala Phe Ala Ile Gly Ala Arg Ala Arg Ala Glu Ala Glu
                325                 330                 335

Gln Met Ala Lys Lys Ala Glu Ala Phe Gln Leu Tyr Gln Glu Ala Ala
            340                 345                 350

Gln Leu Asp Met Leu Leu Glu Lys Leu Pro Gln Val Ala Glu Glu Ile
        355                 360                 365

Ser Gly Pro Leu Thr Ser Ala Asn Lys Ile Thr Leu Val Ser Ser Gly
    370                 375                 380

Ser Gly Thr Met Gly Ala Ala Lys Val Thr Gly Glu Val Leu Asp Ile
385                 390                 395                 400

Leu Thr Arg Leu Pro Glu Ser Val Glu Arg Leu Thr Gly Val Ser Ile
                405                 410                 415

Ser Gln Val Asn His Lys Pro Leu Arg Thr Ala
            420                 425

## Description

### [Technical Field]

The present invention relates to an Alzheimer's disease biomarker. Specifically, the present invention relates to a biomarker useful for testing Alzheimer's disease and related diseases, and use thereof. The present application claims priority based on Japanese Patent Application No. 2017-108766 filed on May 31, 2017, the entire contents of which are incorporated herein by reference.

### [Background Art]

Deposition of senile plaques made of amyloid beta protein in the brain is the most typical pathological finding of Alzheimer's disease (hereinafter sometimes abbreviated as "AD"), and amyloid deposition or polymer formation is considered to be a cause leading to the onset of AD. Therefore, the regulation of amyloid beta (Aβ) metabolism (production, polymer formation, and degradation/removal) is considered to be a target for prevention/treatment of the disease, and therapeutic drugs have been developed. Recent studies have shown that, in AD, the formation of senile plaques with Aβ aggregates deposited is initiated more than 20 years before onset thereof, and that senile plaques already exist widely in the brain at the time of onset. Eventually, it is thought that, when neurofibrillary tangles mainly composed of Tau protein aggregates appear, dementia develops through mild cognitive impairment (MCI). In response to such study results, the National Institute on Aging (NIA) and the Alzheimer's Association revised the diagnostic criteria in 2011, and have come to advocate the asymptomatic stage of Alzheimer's disease with a change in brain pathology as "Preclinical AD" (NPL 1). In addition, it has been clarified that definitive treatment methods targeting amyloid, such as vaccine therapy, have limited effects after onset. It has come to be considered that treatment should be made not after onset in which senile plaques have already been completed, but before onset when amyloid pathology is progressing. Under such a background, attempts have been made to diagnose and treat AD before onset or at the stage of mild cognitive impairment (MCI), for example, by cerebrospinal fluid examination or amyloid imaging using PET images, and such diagnosis and treatment have entered a technically possible stage. However, cerebrospinal fluid examination involves invasion and is difficult to carry out in general hospitals and clinics. In addition, PET is expensive in terms of equipment and reagents, and, besides, the possibility of radiation damage cannot be completely denied. From this background, development of a simpler, less invasive and inexpensive diagnostic method is expected. On the other hand, dementia includes, in addition to Alzheimer's dementia, dementia with Lewy bodies, vascular dementia and frontotemporal dementia (FTD), and differential diagnosis is necessary for appropriate treatment/prevention thereof. In fact, in elderly patients with depression and the like, differentiation from Alzheimer's dementia is often necessary.

On the other hand, the finding has been reported that exosomes existing in the brain bind to Aβ protein and work to remove them, and the role of exosomes at the time of onset of AD is attracting attention. However, changes in exosome levels in AD have been completely unknown. In this regard, the present inventors' research group has reported a phenomenon that exosome secretion is markedly reduced in cells treated with Aβ1-42 (NPL 2).

### [Citation List]

### [Non Patent Literature]

[NPL 1] Alzheimer's Dementia 7: 263-269, 2011
[NPL 2] Journal of Alzheimer's Disease 53 (2016) 1433-1441

### [Summary of Invention]

### [Technical Problem]

As described above, although there are technologies used for diagnosis of AD, there are many problems to be overcome, and it is desired to diagnose AD with a simple method at a low cost with less invasiveness to patients. In order to meet such a demand, an object of the present invention is to provide a marker useful for early diagnosis and differentiation of AD, and use thereof.

### [Solution to Problem]

In pursuing research for the purpose of solving the problems, the present inventors have focused on flotillin localized in lipid rafts of exosomes and decided to examine whether it can be used as an AD biomarker. First, when the level of flotillin in cerebrospinal fluids of an AD patient was examined, it was found that the level was significantly decreased as compared with that of a non-AD patient. The same tendency was observed for the cerebroventricular fluid. On the other hand, the flotillin level in cerebrospinal fluid of patients with mild cognitive impairment due to AD (MCI due to AD) was significantly lower than that of patients with mild cognitive impairment due to non-AD (MCI due to non-AD). As a result of further examination, a significant decrease in serum flotillin level in AD patients has been surprisingly confirmed, and it has been revealed that flotillin functions as a blood biomarker of AD. Moreover, as a result of verifying the usefulness of flotillin using clinical specimens, it has been demonstrated that the serum flotillin level is a marker highly specific for AD and extremely useful for early detection of AD.

It is known that brain-derived exosomes are present in blood, but their abundance (that is, amount of exosomes to be transferred/excreted from the brain into the blood) has not been clarified. However, since exosomes derived from tissues or organs other than the brain are expected to be much more abundant than those derived from the brain, it is an unexpectable, surprising fact that AD patients and non-AD patients are remarkably different in the blood level of flotillin, which is one of the constituent molecules of exosomes. The above findings can also be said to indicate that brain-derived exosomes are unexpectedly present in blood relatively abundantly, which is also important and interesting.

The following inventions are based mainly on the above results and considerations.
[1] An Alzheimer's disease biomarker composed of blood flotillin.
[2] A test method for Alzheimer's disease using the level of the biomarker according to [1] as an index.
[3] The test method according to [2], which is a test method for early detection of Alzheimer's disease.
[4] The test method according to [3], including the following steps (1) and (2) of:
   (1) detecting the biomarker in a blood specimen; and
   (2) determining the possibility of onset of Alzheimer's disease or the presence or absence of affection of Alzheimer's disease based on the detection result.
[5] The test method according to [2], which is a test method for differentiating Alzheimer's disease.
[6] The test method according to [5], which is used for differentiation from dementia other than Alzheimer's dementia.
[7] The test method according to [6], wherein the dementia other than Alzheimer's dementia is vascular dementia, dementia with Lewy bodies, or frontotemporal dementia.
[8] The test method according to any one of [5] to [7], including the following steps (1) and (2) of:
   (1) detecting the biomarker in a blood specimen; and
   (2) determining whether the affected disease is Alzheimer's disease based on the detection result.
[9] The test method according to [4] or [8], wherein the blood specimen is serum or plasma.
[10] An Alzheimer's disease test reagent including a substance having specific binding to the biomarker according to [1].
[11] The Alzheimer's disease test reagent according to [10], wherein the substance is an anti-flotillin antibody.
[12] An Alzheimer's disease test kit including the Alzheimer's disease test reagent according to [10] or [11].

### [Brief Description of Drawings]

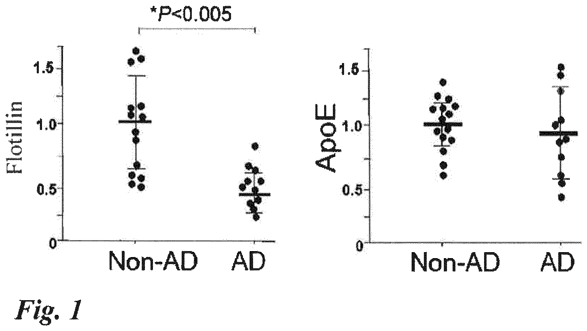

[Fig. 1] Comparison of flotillin levels in the cerebroventricular fluids of autopsied brains. Non-AD: Non-Alzheimer's disease. AD: Alzheimer's disease. Both are based on a definitive diagnosis by a pathological diagnosis. The level of ApoE observed as an unchanged molecule (control) shows no change.

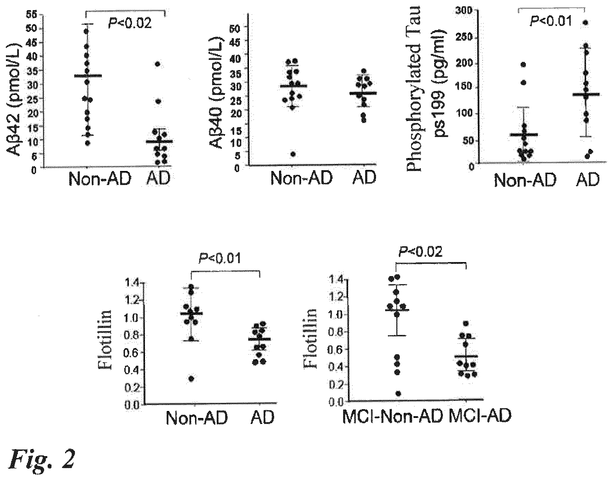

[Fig. 2] Comparison of levels of conventional cerebrospinal fluid markers, Aβ1-42 and phosphorylated Tau protein, in cerebrospinal fluids (upper) and comparison of levels of a novel marker flotillin in cerebrospinal fluids (lower). Non-AD: Non-Alzheimer's disease. AD: Alzheimer's disease. MCI-due to AD (MCI patients who showed amyloid β deposition in PET). MCI-due to non-AD (MCI patients who did not show amyloid β deposition in PET). The flotillin level is significantly lowered in AD and MCI-due to AD than in Non-AD and MCI-due to non-AD, respectively.

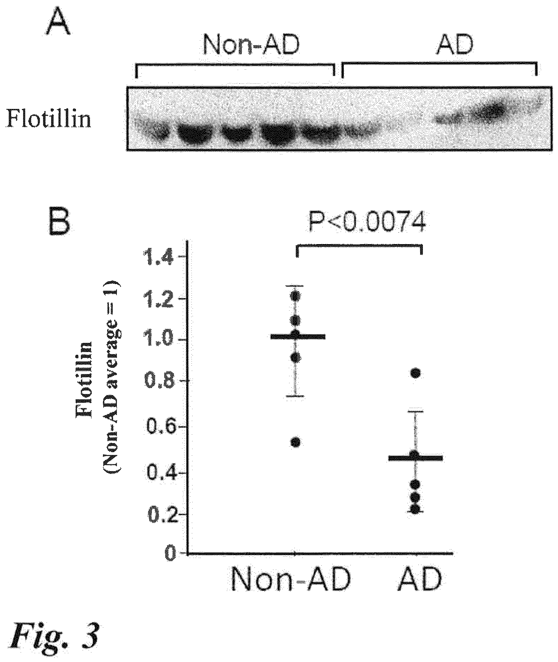

[Fig. 3] Comparison of serum flotillin levels. A shows the result of Western blotting, and B shows the result of ELISA. Non-AD: Non-Alzheimer's disease. AD: Alzheimer's disease. The serum flotillin level was significantly lowered in AD (through a PET test) in comparison with Non-AD (through a PET test). The data is shown as mean ± S.D. A significant difference is calculated by t-test (n = 5).

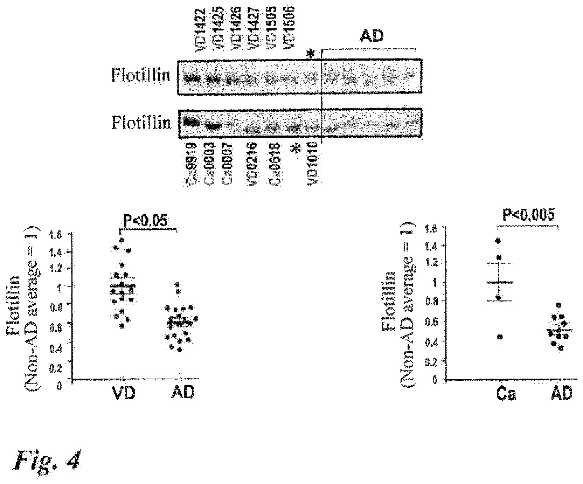

[Fig. 4] Comparison of serum flotillin levels. The serum flotillin levels were compared between AD patients (n = 17) and vascular dementia (VD) patients (n = 20) and between AD patients (n = 4) and cancer patients (n = 10). VD: vascular dementia (the number indicates a sample identification number). Ca: death from cancer (without dementia).

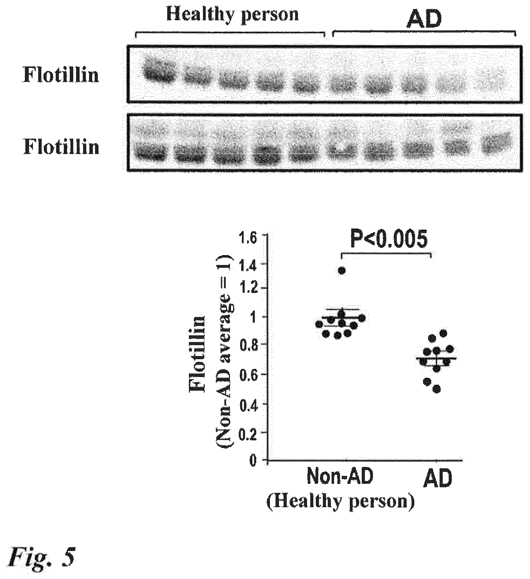

[Fig. 5] Comparison of serum flotillin levels. The serum flotillin levels were compared between AD patients (n = 10) and healthy persons (Non-AD) (n = 10).

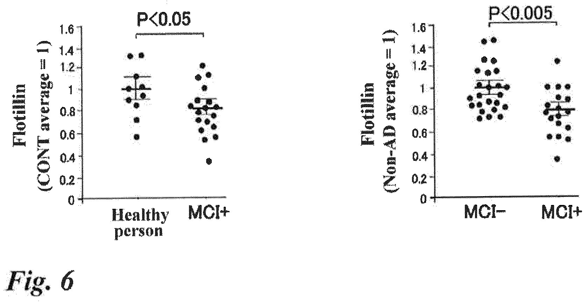

[Fig. 6] Diagnosis of mild cognitive impairment (MCI) based on the serum flotillin level. The serum flotillin levels were compared among healthy persons (n = 10), MCI patients positive for AD pathology (MCI+) (n = 18) and MCI patients negative for AD pathology (MCI-) (n = 24) in a PET test.

### [Description of Embodiments]

### 1. Alzheimer's disease (AD) biomarker

A first aspect of the present invention relates to an Alzheimer's disease biomarker. As used herein, the "Alzheimer's disease biomarker" refers to a biomolecule that serves as an index of the affection or onset of Alzheimer's disease (AD). As will be described later, the biomarker of the present invention is useful for early detection of AD and differentiation of AD. The biomarker of the present invention is used for, for example, tests on a person who is likely/suspected to develop or suffer from AD (suspected AD patient, potential AD patient).

As used herein, the "biomolecule" refers to a molecule (compound) found in a living body. Flotillin, which is a biomolecule, is used as a biomarker in the present invention, and, in the use thereof (typically, application thereof to a test method for AD), the molecule in a sample/specimen separated from a living body is used.

The biomarker of the present invention is composed of blood flotillin. Flotillin is a protein localized in lipid rafts of cells, and is also known as an exosome marker. Utilizing the characteristic, flotillin is used for the detection of exosomes in cerebrospinal fluid and blood, for research on lipid rafts, and the like. A typical amino acid sequence of flotillin is shown in SEQ ID NO: 1 (GenBank, ACCESSION: NP_005794, DEFINITION: flotillin-1 isoform 1 [Homo sapiens]). As for flotillin, there are reports such as Hulsbusch N et al, European Journal of Cell Biology. 2015; 94 (11): 531-45, Li Z, et al, American Journal of Cancer Research. 2016; 6 (1): 38-50.

As used herein, the wording "(in) blood" means existing in blood. Therefore, the biomarker composed of blood flotillin according to the present invention is detected in blood specimens (serum, plasma, and the like).

### 2. Test for Alzheimer's disease (AD)

A second aspect of the present invention relates to use of the biomarker of the present invention, and provides a test method for Alzheimer's disease (AD) (hereinafter also referred to as "test method of the present invention"). The test method of the present invention is useful as a means for early detection of AD. The test method of the present invention is also useful as a means for differentiation of AD. According to the test method of the present invention, an objective basis for enabling determination of the possibility of onset of AD or the presence or absence of affection, or differentiation of AD is provided. The information (test results) provided by the test method of the present invention is based on an objective index called biomarker (biomolecule), which enables the determination and evaluation of the possibility of onset of AD by itself. However, it may be used as an auxiliary to make a final decision (typically, a definitive diagnosis) in consideration of any other index as necessary.

### 2-1. Early detection of Alzheimer's disease (AD)

A first embodiment of the test method of the present invention is intended for early detection of AD, and enables detection of AD before the onset of AD or at an early stage of AD. That is, according to the test method of the present invention, an objective basis for deciding the possibility that a test subject (typically, a person who is likely to develop or suffer from AD) will develop AD in the future or the presence or absence of affection of AD is presented.

Typically, with respect to cognitive impairment, an asymptomatic stage or a stage where mild cognitive impairment (MCI) is observed corresponds to "before onset of AD". The "mild cognitive impairment" is a stage where a person has a complaint of forgetfulness, and has been confirmed to have memory disorder for his/her age through neuropsychological tests, but is normal in general cognitive function and activities of daily living, and does not suffer from dementia. On the other hand, the "early stage of AD" refers to a state where a person has memory disorder, disorientation, troubles with judgment and problem-solving ability and social and instrumental activities of daily living, which are caused by impaired cognitive function, but has no trouble with basic activities of daily living.

In the test method of the present invention, the level of the biomarker of the present invention in a blood specimen derived from a subject (that is, blood flotillin level) is used as an index. The "level" as used herein typically means "amount" or "concentration". However, the term "level" is used also to indicate whether or not the molecule to be detected can be detected (i.e., apparently present or not) in accordance with common practices and common technical knowledge.

Typically, the test method of the present invention includes the following steps (1) and (2):
(1) detecting the biomarker of the present invention in a blood specimen; and
(2) determining the possibility of onset of Alzheimer's disease or the presence or absence of affection of Alzheimer's disease based on the detection result.

### Step (1)

In step (1), a blood specimen collected from a subject is prepared to detect the biomarker of the present invention, that is, blood flotillin. It is not essential to accurately quantify the level of blood flotillin. That is, it is only necessary to detect the level of blood flotillin to an extent such that a desired determination can be made in the subsequent step (2). For example, the detection can also be performed so that it can be determined whether or not the blood flotillin level in the specimen exceeds a predetermined reference value.

As the blood specimen, serum, plasma, whole blood, and the like can be used. Preferably, serum or plasma is used. Preparation of serum from whole blood can be performed by a conventional method (typically, coagulation by standing at normal temperature and subsequent centrifugation). The same applies to the preparation of plasma (typically, addition of a coagulant and subsequent centrifugation),

The subject is not particularly limited. That is, the present invention can be widely applied to those who need to be determined in terms of the possibility of onset of Alzheimer's disease or the presence or absence of affection of Alzheimer's disease. Typically, the subject is a person who is likely to develop or suffer from Alzheimer's disease. For example, those who have mild cognitive impairment, those who have a genetic risk such as having a relative with Alzheimer's disease, those who have been confirmed to have a risk of developing or suffering from Alzheimer's disease by any other test, and the like are suitable subjects.

The method for detecting blood flotillin is not particularly limited, but preferably an immunological technique is used. According to the immunological technique, blood flotillin can be detected quickly with high sensitivity. Also, the operation is simple. In the detection of blood flotillin by the immunological technique, a substance having specific binding to flotillin is used. As the substance, an anti-flotillin antibody is usually used. However, any substance can be used, without limitation to the anti-flotillin antibody, so long as the substance has specific binding to flotillin and the amount thereof binding to flotillin can be measured. Flotillin has already been purified and identified, and an antibody exhibiting specific binding to flotillin can be prepared by a conventional method. Anti-flotillin antibodies are sold by various companies (for example, Flotilin-1 antibody and Flotillin-2 antibody which are products of CST1 Japan, Inc., Flotillin1 antibody and Flotillin-2 antibody which are products of Abcam pic., and flotillin antibodies which are products of Santa-Cruz Biotechnology, Inc.), and these commercially available anti-flotillin antibodies can also be used.

Examples of the measurement method can include latex coagulating method, fluorescence immunoassay method (FIA method), enzyme immunoassay method (EIA method), radioimmunoassay method (RIA method), and Western blotting method. Preferable measurement methods can include FIA method and EIA method (including ELISA method). These methods enable quick and simple detection with high sensitivity. In the FIA method, a fluorescently labeled antibody is used to detect an antigen-antibody complex (immune complex) using fluorescence as a signal. On the other hand, in the EIA method, an enzyme-labeled antibody is used to detect an immune complex using, as a signal, color development or luminescence based on an enzyme reaction. Each measurement method can be performed by a standard protocol, but those skilled in the art can easily improve some of the conditions and procedures according to the detection purpose.

The ELISA method has many advantages such as high detection sensitivity, high specificity, excellent quantitativeness, simple operation, and suitability for simultaneous processing of multiple specimens. An example of a specific operation method when using the ELISA method will be shown below. First, an anti-flotillin antibody is immobilized on an insoluble support. Specifically, for example, the surface of a microplate is sensitized (coated) with an anti-flotillin antibody. A blood specimen is brought into contact with the thus-immobilized antibody. As a result of this operation, if the antigen (flotillin) against the immobilized anti-flotillin antibody is present in the blood specimen, an immune complex is formed. After removal of non-specific binding components by a washing operation, the immune complex is labeled by adding an antibody to which the enzyme binds, and then the substrate of the enzyme is reacted to cause color development. Then, the immune complex is detected using the amount of the color developed as an index.

Not only a non-competition method but also a competition method (a method in which an antigen is added together with the specimen to cause competition) may be used. Further, either a method of directly detecting flotillin in the specimen with a labeled antibody or a sandwich method may be employed. In the sandwich method, two types of antibodies having different epitopes (capture antibody and detection antibody) are used. The ELISA method is detailed in many books and papers which can be referred to when setting the experimental procedures and experimental conditions of each method.

### Step (2)

In step (2), the possibility of onset of Alzheimer's disease or the presence or absence of affection of Alzheimer's disease is determined based on the detection result in step (1). In order to enable accurate determination, it is preferable to make determination after comparison of the detected value obtained in step (2) with the detected value of the control specimen (control). For example, the biomarker level of a healthy person can be used as the control.

As will be shown in the Examples below, the level of the biomarker of the present invention is low in AD patients and patients with mild cognitive impairment due to AD. That is, the level of the biomarker of the present invention and the onset/affection of AD show a negative correlation. Therefore, basically, a low detected value of the biomarker serves as an index of a high possibility of onset of Alzheimer's disease. The same applies to the determination of the presence or absence of affection, and a low detected value of the biomarker serves as an index of affection of Alzheimer's disease.

Mild cognitive impairment (MCI) is a state in which a person has impairment in a part of cognitive function but has no trouble with daily life, and is positioned as an intermediate stage between a healthy state and dementia. In general, MCI is broadly classified into mild cognitive impairment due to AD (MCI due to AD) and mild cognitive impairment due to non-AD (MCI due to non-AD). If a patient with mild cognitive impairment is a subject in the test method of the present invention, the determination of the presence or absence of affection of Alzheimer's disease is determination as to whether the subject's mild cognitive impairment is due to AD (MCI due to AD) or not due to AD (MCI due to non-AD). According to the test method of the present invention, the presence or absence of mild cognitive impairment due to AD is determined based on an objective index, and early therapeutic intervention can be performed for a person who has been determined to suffer from mild cognitive impairment due to AD. In mild cognitive impairment, early and appropriate treatment or prevention may improve symptoms, or progression thereof may be prevented or delayed. The test method of the present invention provides an objective basis (validity) of early therapeutic intervention for mild cognitive impairment, is significant from the viewpoint of preventive medicine, and contributes to the maximization of the therapeutic effect.

The determination in step (2) may be qualitative, semi-quantitative, or quantitative. Examples of qualitative determination and quantitative determination will be shown below. The number of determination sections, the level of the biomarker associated with each determination section, determination results, and the like can be arbitrarily set through preliminary experiments and the like, without being bound by the following examples. Further, the reference value and cut-off value used for the determination may be set in consideration of, for example, the specimen to be used and the required accuracy (reliability). In setting the reference value and cut-off value, statistical analysis using a large number of specimens may be used. It should be noted that the determination here can be automatically/mechanically made without depending on the decision made by a person having specialized knowledge such as a doctor or a laboratory technician, as is apparent from the decision criteria.

### (Example 1 of qualitative determination when determining possibility of onset)

When the detected value (flotillin amount) is lower than the reference value, the subject is determined as "being highly likely to develop AD". When the measured value is higher than the reference value, the subject is determined as "being less likely to develop AD".

### (Example 2 of qualitative determination when determining possibility of onset)

When no reactivity is observed (negative), the subject is determined as "being highly likely to develop AD". When the reactivity is observed (positive), the subject is determined as "being less likely to develop AD".

### (Example 1 of qualitative determination when determining presence or absence of affection)

When the detected value (flotillin amount) is lower than the reference value, the subject is determined as "suffering from AD". When the measured value is higher than the reference value, the subject is determined as "not suffering from AD".

### (Example 2 of qualitative determination when determining presence or absence of affection)

When no reactivity is observed (negative), the subject is determined as "suffering from AD". When the reactivity is observed (positive), the subject is determined as "not suffering from AD".

### (Example of quantitative determination when determining possibility of onset)

As will be shown below, the possibility of onset (%) is set in advance for each range of detected values, and is determined from the detected value.
Detected value < a: the probability of onset of AD is greater than 80%
a ≤ Detected value < b: the possibility of onset of AD is 20% to 80%
b < Detected value: the possibility of onset of AD is less than 20%

In this example, the detected values are divided into three sections, but the number of sections can be set arbitrarily. An example of the number of sections is 2 to 10, preferably 2 to 5.

### (Example of quantitative determination when determining presence or absence of affection)

As will be shown below, the possibility of affection (%) is set in advance for each range of detected values, and is determined from the detected value.
Detected value < a: the probability of affection of AD is greater than 80%
a ≤ Detected value < b: the possibility of affection of AD is 20% to 80%
b < Detected value: the possibility of affection of AD is less than 20%

In this example, the detected values are divided into three sections, but the number of sections can be set arbitrarily. An example of the number of sections is 2 to 10, preferably 2 to 5.

In one embodiment of the present invention, the detected value at a certain time point and a past-detected value are compared for the same subject to examine the presence/absence of increase/decrease in biomarker level and/or the degree of such increase/decrease. The resulting data on the change in biomarker level is useful information for monitoring the fluctuation in possibility of onset or the presence or absence of affection, or for grasping the preventive/therapeutic effect. Specifically, for example, based on the fluctuation in biomarker level, it can be determined that the possibility of onset has increased or decreased or is unchanged in a period between the previous test and the current test. If such evaluation is performed in parallel with prevention or treatment, the preventive/therapeutic effect can be confirmed, and, besides, signs of the onset or progress of AD can be grasped.

### 2-2. Differentiation of Alzheimer's disease (AD)

A second embodiment of the test method of the present invention enables differentiation between Alzheimer's dementia and non-Alzheimer's dementia. That is, according to the test method of this embodiment, there is indicated an objective basis for identifying whether the dementia suffering from the test subject (typically, a patient with dementia) is Alzheimer's dementia or any other dementia. Specifically, according to the test method of the present invention, it is possible to obtain a determination result of "being Alzheimer's dementia" or "being dementia other than Alzheimer's dementia" regarding the affected disease. The present invention can be used for differentiation from one or more specific dementias (dementias other than Alzheimer's dementia). In that case, for example, it is determined that the affected disease is "Alzheimer's dementia" or "a specific dementia other than Alzheimer's dementia". Specific examples of the specific dementias herein include vascular dementia, dementia with Lewy bodies, and frontotemporal dementia. Therefore, in the test method of the present invention, for example, differentiation is made between Alzheimer's dementia and any one or more of these dementias.

Also in the test method of this embodiment, the level of the biomarker of the present invention (i.e., blood flotillin level) in a blood specimen derived from a subject is used as an index, and, typically, the following steps (1) and (2) are performed:
(1) detecting the biomarker of the present invention in a blood specimen; and
(2) determining whether the affected disease is Alzheimer's disease based on the detection result.

Since step (1) can be performed in the same manner as step (1) of the above-described embodiment (early detection of AD), the above description is applied to omit redundant description. However, in this embodiment, normally, the subject is a person exhibiting a symptom that can be said to be characteristic of AD (e.g., mild memory disorder), and is likely/suspected to develop AD (suspected AD patient), a patient with depression, and a patient with any other dementia such as dementia with Lewy bodies, or frontotemporal dementia.

### Step (2)

In step (2), based on the detection result in step (1), it is determined whether or not the affected disease is AD. In order to enable accurate determination, it is preferable to make determination after comparison of the detected value obtained in step (2) with the detected value of the control specimen (control). Usable controls include a biomarker level of an AD patient (positive control), a biomarker level of a patient with dementia other than AD (negative control), and a biomarker level in a patient with spinocerebellar degeneration, motor neuron disease or multiple sclerosis (negative control).

As will be shown in the Examples below, the level of the biomarker of the present invention is low in AD patients. That is, the level of the biomarker of the present invention and the affection of AD show a negative correlation. Therefore, basically, a low detected value of the biomarker serves as an index of AD.

The determination may be qualitative, semi-quantitative, or quantitative. Examples of qualitative determination and quantitative determination will be shown below. The number of determination sections, the level of the biomarker associated with each determination section, determination results, and the like can be arbitrarily set through preliminary experiments and the like, without being bound by the following examples. The reference value and cut-off value used for the determination may be set in consideration of, for example, the specimen to be used, the target to be differentiated, and the required accuracy (reliability). In setting the reference value and cut-off value, statistical analysis using a large number of specimens may be used. It should be noted that the determination here can be automatically/mechanically made without depending on the decision made by a person having specialized knowledge such as a doctor or a laboratory technician, as is apparent from the decision criteria.

### (Example of qualitative determination)

When the detected value (flotillin amount) is lower than the reference value, the affected disease is determined as "AD" or "being highly likely to be AD". When the detected value is higher than the reference value, the affected disease is determined as "dementia other than AD" or "being highly likely to be dementia other than AD". When the detected value is higher than the reference value, the affected disease can also be determined as "a specific dementia other than AD" or "being highly likely to be a specific dementia other than AD".

### (Example of quantitative determination)

As will be shown below, the "possibility of AD (%)" is set in advance for each range of detected values, and is determined from the detected value.
Detected value < a: the possibility of AD is greater than 80%
a ≤ Detected value < b: the possibility of AD is 20% to 80%
b < Detected value: the possibility of AD is less than 20%

In this example, the detected values are divided into three sections, but the number of sections can be set arbitrarily. An example of the number of sections is 2 to 10, preferably 2 to 5.

### 3. Test reagent and test kit

The present invention further provides a test reagent and a test kit that can be used in the test method of the present invention. The reagent of the present invention is composed of the biomarker of the present invention, that is, the substance having specific binding to blood flotillin. A specific example of the substance is an anti-flotillin antibody, but any substance can be used in addition to the anti-flotillin antibody, as long as the substance exhibits specific binding to blood flotillin and can be used in the test method of the present invention.

The type and origin of the anti-flotillin antibody are not particularly limited as long as it has specific binding to flotillin. Further, any of a polyclonal antibody, an oligoclonal antibody (a mixture of several to several tens of antibodies), and a monoclonal antibody may be used. As the polyclonal antibody or oligoclonal antibody, an anti-serum-derived IgG fraction obtained by animal immunization and, additionally, an antibody affinity-purified using an antigen can be used. The anti-flotillin antibody may be an antibody fragment such as Fab, Fab', F(ab')₂, scFv, or dsFv antibody.

Anti-flotillin antibody may be prepared in a common procedure. If a commercial product is available, the commercial product may be used. For example, an antibody may be prepared by an immunological method, a phage display method, or a ribosome display method. The preparation of a polyclonal antibody by an immunological method may be carried out by the following procedure. An antigen (flotillin or its portion) is prepared, and an animal such as a rabbit is immunized using the antigen. An antigen is obtained by purification of a living body sample. Alternatively, a recombinant antigen may be used. The recombinant antigen can be prepared by, for example, a gene (or a portion of the gene) coding flotillin is introduced into an appropriate host using a vector, and expressed in the recombinant cell thus obtained.

In order to enhance the immunological induction action, an antigen bound with a carrier protein may be used. Examples of the carrier protein include KLH (Keyhole Limpet Hemocyanin), BSA (Bovine Serum Albumin), and OVA (Ovalbumin). Binding between the antigen and the carrier protein can use, for example a carbodiimide method, a glutaraldehyde method, a diazo condensation method, or an MBS (maleimide benzoyloxy succinimide) method. On the other hand, an antigen prepared by expressing flotillin (or its portion) as a fused protein with GST, β-galactosidase, maltose-bound protein, or histidine (His) tag can be used. The fused protein can be purified simply by a general-purpose method.

As necessary, immunization is repeated, the blood is collected after the antibody titer is thoroughly increased, and the blood serum is obtained by, for example, centrifugation treatment. The antiserum thus obtained is subjected to affinity purification, and thus obtaining a polyclonal antibody.

On the other hand, the monoclonal antibody may be prepared by the following procedure. Firstly, immunization operation is carried out by the above-described procedure. As necessary, immunization is repeated, and antibody-producing cells are extracted from the immunized animal after the antibody titer is thoroughly increased. Secondly, the antibody-producing cells thus obtained and myeloma cells are fused to obtain hybridoma. Subsequently, the hybridoma is cloned from a single cell, and then a clone producing an antibody having high specificity to flotillin is selected. The culture solution of the selected clone is purified to obtain the desired antibody. Alternatively, the hybridoma is proliferated to the desired number or more, and then the cells are transplanted into the abdominal cavity of an animal (for example, mouse), and proliferated in the ascites fluid, and the ascites fluid is purified to obtain the desired antibody. Affinity chromatography using, for example, protein G, protein A is suitable for the purification of the above-described culture solution or ascites fluid. Alternatively, affinity chromatography including an immobilized antigen (flotillin) may be used. Other method such as ion exchange chromatography, gel filtration chromatography, ammonium sulfate fractionation, or centrifugation may be used. These methods may be used alone or in combination.

Providing that the specific binding properties to flotillin are maintained, the antibody thus obtained may be subjected to various modifications. The modified antibody may be used as the reagent in the present invention.

When the anti- flotillin antibody is a labeling antibody, the amount of bound antibody can be directly detected using the amount of labeling as the index. Accordingly, the test method is more simplified. On the other hand, there are problems that an antibody bound with a labeling agent must be prepared, and that the detection sensitivity is commonly low. Therefore, an indirect detection method such as a method using a secondary antibody bound to a labeling agent, or a method using a polymer bound to a secondary antibody and a labeling agent is preferred. The secondary antibody herein is an antibody having specific binding properties to the antibody specific to the anti- flotillin antibody. For example, when an antibody specific to the anti- flotillin antibody is prepared as a rabbit antibody, an anti-rabbit IgG antibody may be used as a secondary antibody. Labeled secondary antibodies usable for various types of antibodies such as rabbit, goat, and mouse antibodies are commercially available (for example, Funakoshi Co., Ltd. and Cosmo Bio Co., Ltd.), and suitable one may be appropriately selected according to the reagent in the present invention.

Examples of labeling substances include fluorescent dyes such as fluorescein, rhodamine, Texas red, and Oregon green; enzymes such as horseradish peroxidase, micro-peroxidase, alkaline phosphatase, and β-D-galactosidase; chemiluminescent or bioluminescent compounds such as luminol and acridine dye; radioisotopes such as ³²P, ¹³¹I and ¹²⁵I; and biotin.

In one embodiment, the reagent in the present invention is solid-phased according to the intended use. The insoluble support used for solidification is not particularly limited. For example, an insoluble support made of water-insoluble substance such as a resin including a polystyrene resin, a polycarbonate resin, a silicon resin, and a nylon resin, or glass. The insoluble support carries an antibody by physical adsorption or chemical adsorption.

The kit of the present invention includes the reagent of the present invention as a main component. The kit may further include other reagent used for carrying out the assay (for example, a buffer solution, a blocking reagent, an enzyme substrate, and a color-producing reagent) and/or an apparatus or instrument (for example, a container, a reaction apparatus, and a fluorescence reader). In addition, the kit preferably includes the flotillin as the standard sample. In addition, usually, an instruction manual is attached to the kit of the present invention.

### [Examples]

### <Identification of novel AD marker>

The present inventors' research group has found a phenomenon that exosome secretion is markedly reduced in cells treated with Aβ1-42, from the previous basic research (NPL 1). In the brain with Alzheimer's disease, the Aβ level is remarkably high, and thus the exosome secretion level may also be reduced. Therefore, the level of flotillin used as an exosome marker was analyzed using patient samples.

### 1. Target and method

The following samples were analyzed. The analysis of the samples was approved by the Ethical Review Committees of Nagoya City University, Oita University, and Fukushimura Hospital.
(i) Cerebroventricular fluid of a person diagnosed as having Alzheimer's disease (AD) at necropsy and cerebroventricular fluid of a person diagnosed as having no AD in a similar manner (freeze-stored at -80°C)
(ii) Cerebrospinal fluid of a person diagnosed as having AD in a clinical diagnosis (with an amyloid PET test), and cerebrospinal fluid of a person diagnosed as having spinocerebellar degeneration in a similar manner (freeze-stored at -80°C)
(iii) Cerebrospinal fluid of a person with mild cognitive impairment who was positive for amyloid deposition (MCI due to Alzheimer's disease (AD)) in a clinical diagnosis (with an amyloid PET test), and cerebrospinal fluid of a person with mild cognitive impairment who was negative for amyloid deposition (MCI-due to non-AD) in a similar manner (freeze-stored at -80°C)
(iv) Serum of a person diagnosed as having AD in a clinical diagnosis (with an amyloid PET test), and serum of a person diagnosed as having motor neuron disease in a similar manner (freeze-stored at -80°C)

Western blotting analysis using an anti-flotillin antibody was performed using the above samples. Cerebrospinal fluids and cerebroventricular fluids were each mixed in an equal volume of a sampling buffer (composition: 100 mM Tris = HCL (pH 7.4), 10% glycerol, 4% SDS, 10% mercaptoethanol, and 0.01% bromophenol blue), and the mixture was boiled at 90°C for 5 minutes. Each sample was electrophoresed on 10% Tris-Tricine SDS-PAGE, and then transferred to a PVDF membrane (Immobilon membrane, Millipore).

The PVDF membrane was blocked with a skim milk solution and then incubated in a solution containing a primary antibody at 4°C for 8 to 10 hours. A rabbit polyclonal anti-flotillin antibody (Sigma-Aldrich) was used as the primary antibody. For detection, Aβ1-40- and Aβ1-42-specific ELISA kits (Wako Pure Chemical Industries, Ltd.) were used. The next morning, the PVDF membrane was washed with a PBS-T solution to remove non-specifically bound primary antibodies, and then incubated at room temperature for 1 hour in a solution in which a secondary antibody was dissolved. Thereafter, after thoroughly washing with PBS-T, a reaction was caused with a color developing solution, and a flotillin band was detected with an image analyzer. The software installed in the image analyzer was used for quantification of the intensity of these bands to create a graph, and statistical analysis was made. In addition, the Aβ1-40 and Aβ1-42 concentrations of some of cerebrospinal fluids were quantified with the ELISA kits.

### 2. Results

Western blotting analysis was performed to evaluate the exosome levels in the cerebroventricular fluids and cerebrospinal fluids from the autopsied brains with AD, in terms of the flotillin level as the exosome marker. As a result, as compared with non-AD patients, patients with a definitive diagnosis of AD at necropsy had a markedly decreased flotillin level in the cerebroventricular fluid (Fig. 1).

In addition, the flotillin level in cerebrospinal fluid was significantly decreased in AD patients and patients with mild cognitive impairment (MCI) due to AD as compared with that in patients with spinocerebellar degeneration and MCI due to non-AD, respectively (lower in Fig. 2). In addition, it was confirmed that, in cerebrospinal fluid samples of AD patients, a decrease in Aβ1-42 and an increase in phosphorylated tau, as conventionally known cerebrospinal fluid markers, were observed (upper in Fig. 2), and that the known markers also certainly fluctuated.

As a result of analysis of the blood flotillin level as a further study, it was found that the level of flotillin in serum was also significantly decreased in the AD patients as compared with that in the Non-AD patients (Fig. 3).

### 3. Discussion

The above facts revealed in this study indicate that flotillin (particularly, quantification thereof) is useful as an early diagnostic marker in blood. The flotillin level is not decreased in spinocerebellar degeneration or motor neuron disease, and thus can also be said to be useful for differentiation from other diseases. Furthermore, the flotillin level is decreased (spinal fluid) in the patients with MCI-due to AD, and thus can be expected to be particularly useful for early diagnosis. Serum diagnosis, if possible, can be considered to be easily performed by various persons ranging from practitioners to general hospital doctors because serum collection is relatively safe and simple, and thus can be expected to be useful for early diagnosis and treatment. Moreover, since it can be used relatively inexpensively, it is also advantageous in terms of cost and is expected to lead to a reduction in medical costs.

### <Diagnosis and differentiation using serum flotillin level>

It was verified using clinical specimens that flotillin is useful in differentiating AD from other diseases. The serum flotillin level was shown to be significantly lower in AD patients (AD) than in patients with vascular dementia (VD) (left in Fig. 4). The same tendency was observed between AD patients (AD) and cancer patients (Ca) (right in Fig. 4).

On the other hand, when the serum flotillin level was compared between AD patients and healthy persons, the AD patients (AD) had a significantly lower serum flotillin level than the healthy persons (Non-AD) (Fig. 5).

The serum flotillin levels were compared among healthy persons, MCI patients positive for AD pathology (PET-positive MCI) in a PET (positron emission tomography) test, and MCI patients negative for AD pathology (PET-negative MCI) in a PET test. As a result, the serum flotillin level was significantly reduced in the PET-positive MCI group (MCI+) as compared with that in the healthy group (left graph in Fig. 6). The serum flotillin level was significantly lower in the PET-positive MCI group (MCI+) than in the PET-negative MCI group (MCI-). Thus, it was demonstrated that the MCI patients positive for AD pathology by a PET test can be distinguished from the healthy persons and MCI patients negative for AD pathology by a PET test. That is, it was suggested that the serum flotillin level is useful for early detection of AD.

As described above, it was supported that the serum flotillin level is a marker highly specific for AD and extremely useful for early detection of AD.

### [Industrial Applicability]

The present invention provides a biomarker useful for detection, diagnosis, differentiation, and the like of AD. The biomarker of the present invention is composed of a molecule found in blood. A test method using the biomarker is simple and non-invasive and has excellent practicality. Although there are currently methods for treatment of AD, the effects obtained thereby are limited after onset of AD. It is desirable to start treatment as early as possible. According to the test method of the present invention, the possibility of onset of AD in the future can be determined at a stage before the onset of AD. The determination results are useful information for diagnosis of AD, and are useful for earlier and more appropriate determination of treatment policies (for example, selection of effective prevention/treatment method). By using the test result of the present invention, it is possible to start treatment at an early stage, and to avoid or delay the onset of AD, or to reduce symptoms after onset of AD. Thus, the test method of the present invention greatly contributes to the prevention/treatment of AD.

The present invention is not limited to the above description of the embodiments and examples of the present invention at all. Various modifications that can be easily achieved by those skilled in the art without departing from the claims also fall within the scope of the present invention. The contents of the articles, patent laid-open publications, patent publications, and the like specified herein shall be cited by incorporation in their entity.

## Claims

1. An Alzheimer's disease biomarker comprising blood flotillin.

2. A test method for Alzheimer's disease using the level of the biomarker according to claim 1 as an index.

3. The test method according to claim 2, which is a test method for early detection of Alzheimer's disease.

4. The test method according to claim 3, comprising the following steps (1) and (2) of:
(1) detecting the biomarker in a blood specimen; and
(2) determining a possibility of onset of Alzheimer's disease or presence or absence of affection of Alzheimer's disease based on a detection result.

5. The test method according to claim 2, which is a test method for differentiating Alzheimer's disease.

6. The test method according to claim 5, which is used for differentiation from dementia other than Alzheimer's dementia.

7. The test method according to claim 6, wherein the dementia other than Alzheimer's dementia is vascular dementia, dementia with Lewy bodies, or frontotemporal dementia.

8. The test method according to any one of claims 5 to 7, comprising the following steps (1) and (2) of:
(1) detecting the biomarker in a blood specimen; and
(2) determining whether an affected disease is Alzheimer's disease based on a detection result.

9. The test method according to claim 4 or 8, wherein the blood specimen is serum or plasma.

10. An Alzheimer's disease test reagent comprising a substance having specific binding to the biomarker according to claim 1.

11. The Alzheimer's disease test reagent according to claim 10, wherein the substance is an anti-flotillin antibody.

12. An Alzheimer's disease test kit comprising the Alzheimer's disease test reagent according to claim 10 or 11.
